# NEUE EUROPÄISCHE PATENTSCHRIFT

(11) **EP 0 879 584 B2**
(45) Veröffentlichungstag und Bekanntmachung des Hinweises auf die Entscheidung über den Einspruch: **06.07.2011**
(45) Hinweis auf die Patenterteilung: 18.08.2004
(21) Anmeldenummer: 98108279.5
(22) Anmeldetag: 06.05.1998
(51) Int. Cl.: A61F 2/06

(54) **Koronarer Stent**
Coronary stent
Extenseur coronaire

(30) Priorität: 20.05.1997 DE 29708879 U
(43) Veröffentlichungstag der Anmeldung: 25.11.1998
(73) Patentinhaber: Abbott Laboratories Vascular Enterprises Limited, Dublin 2 (IE)
(72) Erfinder: Von Oepen, Randolf, Dr. Ing., 72145 Hirrlingen (DE)
(74) Vertreter: Schmitz, Hans-Werner

(56) Entgegenhaltungen:
- EP-A1- 0 830 853
- EP-B1- 0 979 059
- WO-A1-96/03092
- WO-A1-96/39102
- WO-A1-97/32543
- DE-A1- 4 418 336
- DE-U1- 29 702 671
- US-A- 5 354 308
- US-A- 5 449 373

## Beschreibung

Die Erfindung bezieht sich auf einen koronaren Stent gemäß Anspruch 1.

Aus der WO 9603092 A1 ist ein Stent mit einem rohrförmigen Körper bekannt, dessen Wand eine relativ biegefeste Stegstruktur aufweist. Die Stegstruktur weist mehrere zueinander benachbarte, durch Stege begrenzte Zellen auf. Der Körper weist an zumindest einem seiner stirnseitigen Enden eine biegeweiche Spitze auf, die Verbinder aufweist, die nur an jedem zweiten oder dritten Ende des stirnseitigen Stegmusters angreifen und ein weiteres Stegmuster mit dem Körper verbinden. Dieses weitere Stegmuster weist Schenkel mit der gleichen Länge wie die Stegmuster des Körpers auf.

Aus dem Stand der Technik sind unterschiedlichste Ausgestaltungsformen von koronaren Stents vorbekannt. Diese bilden eine Gefäßprothese, die aus körperverträglichem Material besteht. Der Stent bzw. die Stentprothese wird dazu verwendet, Blutgefäße oder aber auch andere Körperöffnungen aufzuweiten und in dem aufgeweiteten Zustand zu halten. Zu diesem Zwecke wird der Stent in einem nicht-expandierten Zustand im Körper des Patienten positioniert und nachfolgend durch geeignete Mittel, beispielsweise einen Ballonkatheter expandiert. Bei dem Expandieren werden die einzelnen Stegbereiche des Stents verformt, so daß dieser dauerhaft in der expandierten Form verbleibt.

Einen Stent der zuvor beschriebenen Art zeigt beispielsweise das Gebrauchsmuster 297 02 671.

Ein weiterer Stent ist aus der DE 44 18 336 A bekannt. Diese Druckschrift zeigt einen Stent, der zur Erreichung einer grösseren Biegefähigkeit und Flexibilität über seine gesamte Längserstreckung eine Wandstruktur aufweist, die rautenförmige Zellen umfaßt und einerseits Verbindungen in Form von Knotenbereichen und andererseits Lücken aufweist.

Aus der WO 96/39102 A ist ein Stent bekannt, der ebenfalls über seine gesamte Länge biegeweich ist und hierzu bei der Ausführungsform gemäß Fig. 5eine gleichmäßige Wandstruktur aufweist, an deren Ende schleifenartige Elemente vorgesehen sind, die ausreichend lang sind, damit sie die Enden von Paaren benachbarter Wandelemente zusammenhalten können, wenn der Stent radial ausgedehnt wird.

Aus der WO 97/32543 A ist schließlich ein Stent mit einer völlig gleichmäßigen Stentstruktur bekannt, bei dem der zentrale Bereich genauso aufgebaut ist, wie die beiden Endbereiche. Daher ist auch dieser Stent über seine gesamte Länge gleichmäßig biegeweich bzw. biegehart.

Bei der Konstruktion von Stents ergibt sich grundsätzlich das Problem, daß diese im nicht-expandierten Zustand einen ausreichend kleinen Durchmesser aufweisen müssen, um in den Körper des Patienten eingebracht und dort positioniert werden zu können. Die Stents müssen dabei eine gewisse Flexibilität längs ihrer Längsachse haben, um den Formen von beispielsweise Blutgefäßen folgen zu können. Bei der Expansion muß der Stent auf einen wesentlich größeren Außendurchmesser ausgeweitet werden. Dies muß durch Verformung der einzelnen Stegbereiche so erfolgen, daß Rißbildungen oder ähnliches vermieden werden.

Der Erfindung liegt die Aufgabe zugrunde, einen Stent der eingangs genannten Art zu schaffen, welcher bei einfachem Aufbau, einfacher Herstellbarkeit und sicherer Anwendbarkeit äußerst flexibel ist und dabei dennoch genügend hohe Radialkräfte aufbringen bzw. diese aufnehmen kann.

Erfindungsgemäß wird die Aufgabe durch die Merkmale des Hauptanspruchs gelöst, die Unteransprüche zeigen weitere vorteilhafte Ausgestaltungen der Erfindung.

Durch die Erfindung wird ein äußerst flexibler Stent geschaffen, der den Gefäßwindungen beim Einsetzten in den Körper eines Patienten gut folgen kann. Da die Spitze des erfindungsgemäßen Stents äußerst biegeweich ist, folgt er beim Positionieren den Gefäßwindungen sehr gut und leicht, da er dem Führungsdraht des Ballonkatheders, auf dem der Stent beim Positionieren montiert ist, wie auf einer Schiene folgt.

Der erfindungsgemäße Stent zeichnet sich somit durch eine sehr hohe Biegeweichheit seiner Spitze aus. Beim Positionieren wird der Stent sozusagen von der Spitze durch das Gefäß gezogen, da diese dem Gefäß aufgrund ihrer Biegeweichheit gut folgen kann.

Die biegeweiche Spitze kann auf unterschiedliche Art und Weise hergestellt werden.

Bei einer besonders bevorzugten Ausführungsform werden am Ende des Stents Verbinder ausgebildet, die in etwa der Form einer Uhrfeder entsprechen. Hierbei greift nur an jedem zweiten oder dritten Ende eines vorzugsweise zick-zack-verlaufenden Stegmuster ein Verbinder an. Bevorzugterweise sollten jedoch zumindestens drei Verbinder vorhanden sein, so daß die Spitze sehr gut an den Stent angekoppelt werden kann. An die Verbinder wird zur endgültigen Ausbildung der Spitze zumindestens ein gewunden verlaufendes Stegmuster angebracht. Dieses nachfolgende Stegmuster (Mäandermuster) weist kurze Schenkel auf, damit es nicht in sich zu hart wird. Bevorzugterweise kann dies dadurch erreicht werden, daß die Anzahl der Zick-Zack-Linien erhöht wird.

Bei einer weiteren besonders bevorzugten Ausführungsform können auch alternierend mehrere Verbinder und kurzschenklige mäandrierende Stegmuster am Ende des Stents angebracht werden.

Obwohl die Form der Verbinder nach Art einer Uhrfeder bzw. nach Art einer Schnecke eine besonders bevorzugte Ausführungsform darstellt, sind grundsätzlich auch andere Verbinder denkbar, die eine biegeweiche Ausführung der Spitze möglich machen.

Der erfindungsgemäße Stent zeichnet sich durch eine Reihe erheblicher Vorteile aus.

Die erfindungsgemäße Anordnung ergibt im nicht-expandierten Zustand zum einen eine ausreichende Festigkeit, zum anderen aber auch eine hohe Flexibilität. Hierbei stellen die Stege oder Stegbereiche der Zellen der Wand des Stents sicher, daß der Stent in einfacher und zuverlässiger Weise expandiert werden kann.

Als besonders günstig hat es sich erwiesen, die gefalteten Stege der ersten Zellen zick-zack-förmig auszugestalten, so daß diese bevorzugterweise einen sich in Umfangsrichtung erstreckenden bandartigen Bereich bilden. Diese bandartigen Bereiche erhöhen die Festigkeit des Stents und sichern auch im expandierten Zustand seine Formbeständigkeit.

Im folgenden wird die Erfindung anhand zweier Ausführungsbeispiele in Verbindung mit der Zeichnung beschrieben.

Dabei zeigt:
Fig. 1 eine schematisch stark vereinfachte Darstellung des Grundaufbaus des erfindungsgemäßen Stents,
Fig. 2 eine Darstellung einer ersten Ausführungsform der Stegstruktur der Wand des Stents im nicht-expandierten Zustand, und
Fig. 3 eine der Fig. 2 entsprechende Darstellung einer zweiten Ausführungsform.

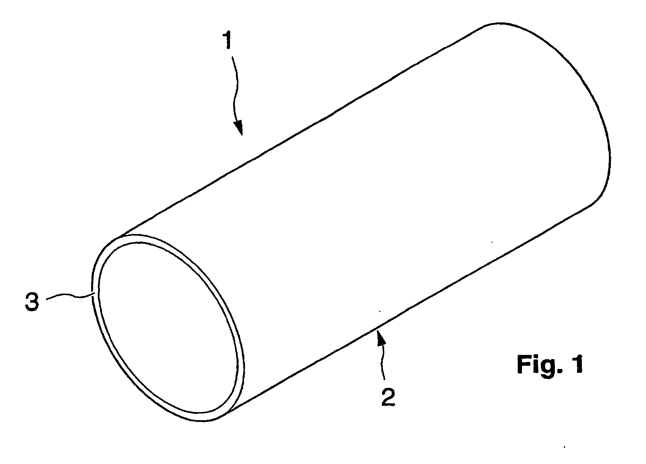

Die Fig. 1 zeigt den grundsätzlichen Aufbau eines erfindungsgemäßen Stents 1, der einen flexiblen, rohrförmigen Körper 2 mit einer Wand aufweist, von der in Fig. 1 die Stirnseite 3 sichtbar ist.

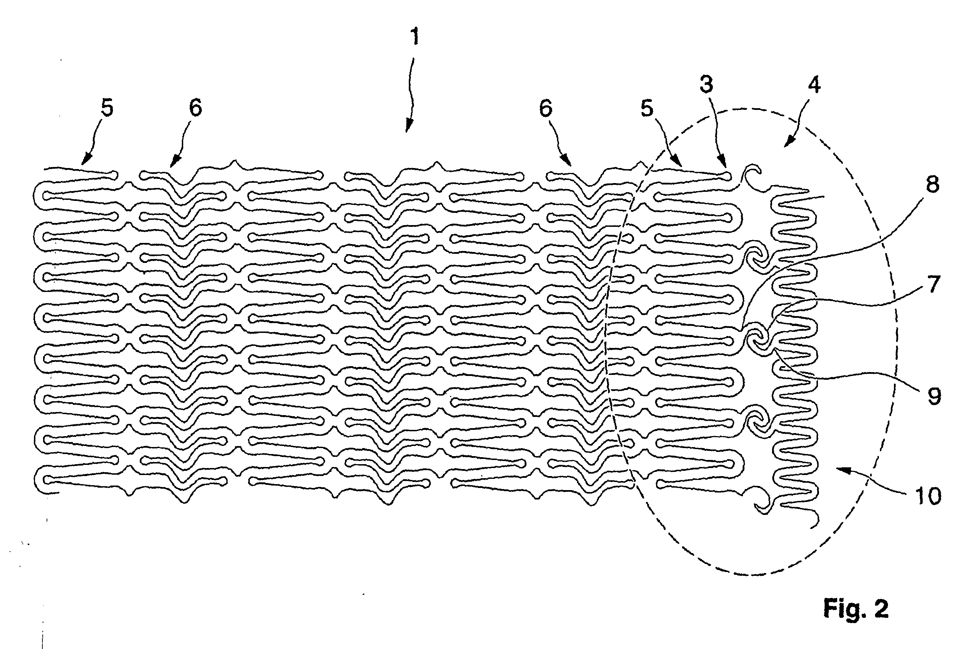

In Fig. 2 ist eine erste Ausführungsform des erfindungsgemäßen Stents dargestellt, wobei zur Erläuterung des Aufbaues des Körpers 2 dessen Wand in planer Darstellung gemäß der Zeichenebene Fig. 2 darstellt ist.

Wie Fig. 2 verdeutlicht, weist der Stent 1 eine Stegstruktur auf, die im Beispielsfalle alternierend Stegmuster 5 und 6 aufweist, wobei die Stegmuster 6 die Stegmuster 5 jeweils miteinander verbinden. Die Stegmuster 5 und 6 weisen zick-zack-förmig verlaufende bzw. mäandrierende Stege auf, die die Zellen der Stegstruktur begrenzen.

Am stirnseitigen Ende 3 des erfindungsgemäßen Stents 1 ist eine biegeweiche Spitze 4 angeordnet. Dieser Bereich ist in Fig. 2 mit einer gestrichelten Linie umrandet.

Bei der in Fig. 2 dargestellten Ausführungsform weist die Spitze 4 eine Mehrzahl von vorzugsweise zumindest 3 Verbindern auf, von den repräsentativ ein Verbinder 7 mit der entsprechenden Bezugsziffer versehen ist. Wie Fig. 2 zeigt, ist der Verbinder ähnlich einer Uhrfeder s-förmig bzw. schneckenförmig gewunden ausgebildet. An einem seiner freien Enden 8 ist der Verbinder 7 mit dem stirnseitigen Stegmuster 5 verbunden. Am anderen freien Ende 9 schließt sich der Verbinder 7 an ein weiteres kurzschenkliges Stegmuster 10 an. Das Stegmuster 10 ist kurzschenkliger als die Stegmuster 5 bzw. 6 ausgebildet. Es verläuft ebenfalls gewunden bzw. zick-zack-förmig und kann somit auch als Mäandermuster bezeichnet werden.

Die Spitze 4 in der zuvor beschriebenen Ausführungsform ist äußerst biegeweich und ermöglicht somit ein Einführen des Stents in den Körper eines Patienten auf äußerst sichere Art und Weise, da die biegeweiche Spitze verhindert, daß sich der Stent in der Gefäßinnenwand verhakt und diese somit verletzt.

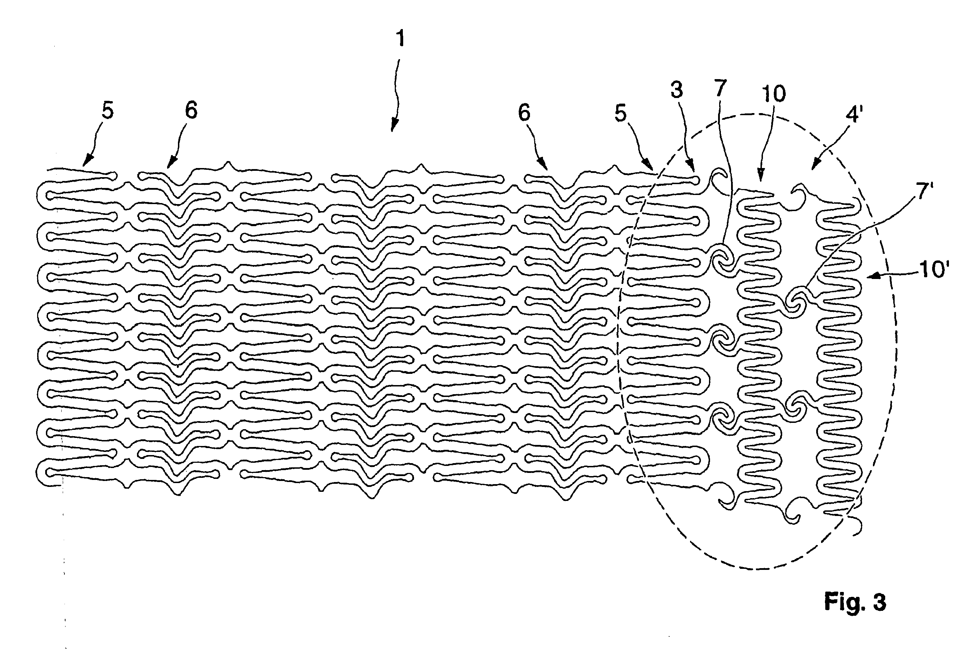

In Fig. 3 ist eine zweite Ausführungsform des erfindungsgemäßen Stents dargestellt. Alle Merkmale, die derjenigen der Fig. 2 entsprechen, sind mit den gleichen Bezugsziffern versehen. Der wesentliche Unterschied zur Ausführungsform gemäß Fig. 2 ist in der Ausführung der biegeweichen Spitze 4' zu sehen, die im Beispielsfalle neben der Mehrzahl von Verbindern 4 und dem an diese angeschlossenen Stegmuster 10 eine weitere Reihe von Verbindern 7' mit einem weiteren daran angeschlossenen Stegmuster 10' aufweist. Fig. 3 verdeutlicht hierbei, daß die Verbinder 7' gegenüber den Verbindern 7 um 180° geklappt angeordnet sind und daß das Stegmuster 10' phasenverschoben zum Stegmuster 10 vorgesehen ist. Ansonsten kann jedoch auf die Ausführung zur Fig. 2 Bezug genommen werden. Auch die Spitze 4' ist äußerst biegeweich und ermöglicht somit ein sicheres Einführen des Stents in den Körper eines Patienten, da dieser den Windungen der Gefäße sehr gut folgt, selbst wenn die Stegstruktur der Wand 2 relativ biegefest ist.

## Patentansprüche

1. Koronarer Stent (1) mit einem rohrförmigen Körper (2), dessen Wand eine relativ biegefeste Stegstruktur aufweist, wobei die Stegstruktur mehrere zueinander benachbarte, durch Stege begrenzte Zellen aufweist, wobei der Körper (2) an zumindestens einem seiner stirnseitigen Enden (3) eine biegeweiche Spitze (4) aufweist, und wobei die Spitze (4) Verbinder (7) aufweist, die an einem ihrer Enden (8) an einem stirnseitigen Stegmuster (5) des Körpers (2) angebracht sind und an ihrem anderen Ende (9) mit einem weiteren gewunden verlaufenden Stegmuster (10) verbunden sind, wobei das weitere Stegmuster (10) im Vergleich zu dem Stegmuster (5, 6) des Körpers (2) kurzschenkliger ausgebildet ist, und nur an jedem zweiten oder dritten Ende des stirnseitigen Stegmusters (5) ein Verbinder (7) angreift.

2. Koronarer Stent nach Anspruch 1, **dadurch gekennzeichnet, dass** die Verbinder (7) S-förmig gewunden ausgebildet sind.

3. Koronarer Stent nach Anspruch 1 oder 2, **dadurch gekennzeichnet, dass** die Verbinder (7) als Federelemente mit hoher Elastizität ausgebildet sind.

4. Koronarer Stent nach einem der Ansprüche 1 bis 3, **dadurch gekennzeichnet, dass** die Spitze (4') zumindestens eine weitere Reihe von Verbindern (7') aufweist, die an dem ersten kurzschenkligen Stegmuster (10) angebracht sind und die an ihrem freien Ende mit einem zweiten kurzschenkligen Stegmuster (10') verbunden sind.

5. Koronarer Stent nach einem der Ansprüche 1 bis 4, **dadurch gekennzeichnet, dass** zumindest drei Verbinder (7, 7') zur Anbringung des kurzschenkligen Stegmusters (10 bzw. 10') vorgesehen sind.

## Claims

1. Coronary stent (1) with a tubular body (2), the wall of which has a relatively bend-resistant crosspiece structure, wherein the crosspiece structure has several cells adjacent to one another and demarcated by crosspieces, wherein the body (2) has a flexible tip (4) on at least one of its frontal ends (3), and wherein the tip (4) has connectors (7) which are attached at one of their ends (8) to a frontal crosspiece pattern (5) of the body (2), and are connected at their other ends (9) to a further winding crosspiece pattern (10), wherein the further crosspiece pattern (10) is constructed with shorter arms compared with the crosspiece pattern (5, 6) of the body (2), and a connector (7) is only linked to every second or third end of the frontal crosspiece pattern (5).

2. Coronary stent according to claim 1, **characterized in that** the connectors (7) are constructed as s-shaped spirals.

3. Coronary stent according to one of claims 1 or 2, **characterized in that** the connectors (7) are constructed as spring elements of high elasticity.

4. Coronary stent according to one of claims 1 to 3, **characterized in that** the tip (4') has at least one further row of connectors (7') which are attached to the first crosspiece pattern (10) with short arms and are connected at their free end to a second crosspiece pattern (10') with short arms.

5. Coronary stent according to one of claims 1 to 4, **characterized in that** at least three connectors (7, 7') are provided for attaching the crosspiece pattern (10 or 10' respectively) with short arms.

## Revendications

1. Extenseur coronaire (1) avec un corps tubulaire (2) dont la paroi présente une structure de type filet relativement rigide à la flexion, la structure de type filet comprenant une pluralité de cellules adjacentes, circonscrites par des nervures, le corps (2) présentant au moins en l'une de ses extrémités frontales (3) un embout ou pointe souple et doux (4), l'embout ou pointe (4) comprenant des éléments d'attache (7) qui, en l'une de leurs extrémités (8) sont fixés à un motif nervuré frontal (5) du corps (2) et, en leur autre extrémité (9) sont reliés à un autre motif nervuré (10) serpentant, d'autre motif nervuré (10) étant réalisé avec des côtés plus courts par comparaison au motif nervuré (5, 6) du corps (2), et un élément d'attache (7) n'étant seulement attaché qu'à chaque deuxième ou troisième extrémité du motif nervuré frontal (5).

2. Extenseur coronaire selon la revendication 1, **caractérisé en ce que** les éléments d'attache (7) sont réalisés de manière sinueuse en forme de S.

3. Extenseur coronaire selon la revendication 1 ou 2, **caractérisé en ce que** les éléments d'attache (7) sont réalisés sous la forme d'éléments à ressort avec une élasticité élevée.

4. Extenseur coronaire selon l'une des revendications 1 à 3, **caractérisé en ce que** l'embout ou pointe (4') comporte au moins une rangée supplémentaire d'éléments d'attache (7'), qui sont fixés au premier motif nervuré à côtés courts (10) et qui, en leur extrémité libre, sont reliés a un second motif nervuré a côtés courts (10').

5. Extenseur coronaire selon l'une des revendications 1 à 4, **caractérisé en ce qu'**au moths trois éléments d'attache (7, 7') sont prévus pour la fixation du motif nervuré à côtés courts (10 ou 10').
